# EUROPÄISCHE PATENTSCHRIFT

(11) **EP 2 637 596 B1**
(45) Veröffentlichungstag und Bekanntmachung des Hinweises auf die Patenterteilung: **28.03.2018**
(21) Anmeldenummer: 11773981.3
(22) Anmeldetag: 30.09.2011
(51) Int. Cl.: A61C 1/08, A61C 8/00, A61B 17/17

(54) **FÜHRUNGSELEMENT UND VORRICHTUNG ZUM EINBRINGEN EINER KNOCHENBOHRUNG**
GUIDE ELEMENT AND DEVICE FOR CREATING A HOLE IN A BONE
ÉLÉMENT DE GUIDAGE ET DISPOSITIF POUR PRATIQUER UN ALÉSAGE D'OS

(30) Priorität: 09.11.2010 AT 18392010
(43) Veröffentlichungstag der Anmeldung: 18.09.2013
(73) Patentinhaber: JEDER GMBH, 1190 Wien (AT)
(72) Erfinder: EDER, Klaus, 2380 Perchtoldsdorf (AT)
(74) Vertreter: Wildhack & Jellinek
(86) Internationale Anmeldenummer: PCT/AT2011/000406
(87) Internationale Veröffentlichungsnummer: WO 2012/061854

(56) Entgegenhaltungen:
- WO-A1-2007/129312
- WO-A1-2010/048648
- US-A1- 2008 319 466
- US-A1- 2010 178 631

## Beschreibung

Die Erfindung betrifft ein Führungselement gemäß dem Oberbegriff des Anspruchs 1 sowie eine Vorrichtung zur durchdringenden Verlängerung einer in hartes Gewebe, insbesondere den Kieferknochen, eingebrachten Sackbohrung gemäß Anspruch 15.

Eine solche Verlängerung einer Knochenbohrung ist beispielsweise im Bereich der Zahnchirurgie bei der Durchführung eines als Sinuslift bezeichneten Eingriffs erforderlich. Als Sinuslift wird eine Operation bezeichnet, bei der die Kieferhöhlenschleimhaut bzw. Sinushaut oder Schneidersche Membran vom Kieferknochen teilweise abgelöst und angehoben wird, um einen Raum zwischen Knochen und Kieferhöhlenschleimhaut herzustellen. In den entstandenen Hohlraum wird dann autologer Knochen, z.B. vom Tuber maxillae, der Linea obliqua, der Kinnregion oder aus dem Beckenkamm (Knochenersatzmaterialien, Knochenspan) bzw. ein synthetisches Knochersatzmaterial, zum Beispiel Knochenersatzmaterial der Marke Bio-Oss® der Firma Geistlich AG, häufig vermischt mit autologem Knochen, eingebracht. Dieses Material soll sich innerhalb von 6 Monaten zu Knochen umbauen, um ein solides Fundament für ein Implantat zu gewährleisten.

Die herkömmliche Vorgehensweise bei einem Sinuslift ist es, im Molarenbereich buccal einen Mucoperiostlappen zu präparieren, und in den dadurch freigelegten Knochen ein ovales Fenster zu fräsen, ohne die darunterliegende Kieferhöhlenschleimhaut zu beschädigen. Die an der Kieferhöhlenschleimhaut hängende, ovale Knochenscheibe wird nun vorsichtig in Richtung der Kieferhöhle gedrückt, wobei gleichzeitig die Kieferhöhlenschleimhaut rund um das Fenster vorsichtig mit speziellen Instrumenten vom Knochen abgelöst wird. Da die Kieferhöhlenschleimhaut sehr zart ist, etwa vergleichbar mit einer Eihaut, ist dieser Vorgang sehr vorsichtig auszuführen, da die Gefahr besteht, die Kieferhöhlenschleimhaut zu beschädigen. Der so in der Kieferhöhle entstandene Raum wird nun durch das Fenster mit dem Knochenersatzmaterial aufgefüllt und das buccale Fenster wird mit einer Folie abgedeckt. Die Folie besteht im Allgemeinen aus einem resorbierbaren Material, wie etwa einer Membran der Marke Bio-Gide® der Firma Geistlich AG. Danach wird der Mucoperiostlappen dicht vernäht. Diese Methode ist verhältnismäßig stark invasiv und belastet den Patienten durch starke Schwellung und Verfärbung bis zu 10 Tage, eventuell auch durch Schmerzen. Dieses Operationsverfahren wird oft auch als "offener" oder "klassischer" Sinuslift bezeichnet. Falls eine ausreichende Restknochenhöhe vorhanden ist, etwa mit einer Höhe von 5 mm, können die Implantate zeitgleich mit dem Sinuslift eingesetzt werden (einzeitiger Sinuslift). Eine volle Belastung der Implantate ist erst nach der Verfestigung des Knochenersatzmaterials möglich. Wenn die Restknochenhöhe zu dünn ist, erfolgt das Einsetzen der Implantate in einem zweiten Eingriff etwa 6-8 Monate nach dem Sinuslift (zweizeitiger Sinuslift).

Ein neueres Verfahren ist der sogenannte crestale Sinuslift, der keine Aufklappung einer Knochenplatte erfordert. Der Zugang zur Kieferhöhle wird vom Kieferkamm her ermöglicht. Dabei wird am zahnlosen Teil des Kieferkamms mittels einer speziell dazu vorgesehenen Stanze (Jesch'sche Stanze) eine Stanzung der Mundschleimhaut bis zum Knochen vorgenommen und mit einer zylindrischen Fräse eine Sackbohrung bis knapp unter die Kieferhöhlenschleimhaut in den Knochen gefräst. Die dabei verwendete Stanze hebt automatisch die Schleimhautscheibe vom Knochen ab und macht eine zentrale Körnung für die weitere Bohrung bzw. Fräsung. Die Bohrung wird im allgemeinen antral, also vom Kamm her, mittels einer Zylinderfräse, beispielsweise mit einem Durchmesser von 3,5 mm, bis ca. 1 mm unter den knöchernen Kieferhöhlenboden gefräst, wobei die Knochendicke zuvor mittels Röntgen gemessen wird. Da die Kieferhöhlenschleimhaut nicht durch die Fräse beschädigt werden darf, darf der Kieferknochen nicht mit dem Fräser ganz durchbohrt werden, sodass am Boden der Sackbohrung eine dünne Knochenplatte verbleibt, an deren Rückseite die Kieferhöhlenschleimhaut anliegt. Herkömmlicherweise wird diese dünne Knochenplatte dann mit einem zylindrischen Instrument vorsichtig in Richtung Kieferhöhle gestoßen, sodass sie mit der Kieferhöhlenschleimhaut, die oberhalb der Knochenscheibe an dieser haftet, in Richtung Kieferhöhle hineingepresst wird. Dieses "Durchstoßen" der Knochenscheibe stellt für den Eingriff einen kritischen Punkt dar, da ein zu festes Hineindrücken der Knochenscheibe dazu führt, dass die Kieferhöhlenschleimhaut zeltförmig angehoben und gespannt wird, wodurch sie beschädigt werden könnte. Die Kieferhöhlenschleimhaut wird dann vorsichtig angehoben, wonach das Knochenersatzmaterial über die Bohrung in den neu geschaffenen Freiraum eingebracht wird. Das Implantat wird danach meist direkt in der Bohrung verankert.

Auch wenn bereits sehr fortschrittliche Verfahren entwickelt worden sind, um die Kieferhöhlenschleimhaut durch die kleine Bohrung, die meist einen Durchmesser von nur etwa 4 mm aufweist, hindurch möglichst schonend und ausreichend weit vom Kieferknochen zu lösen, bleibt der Augenblick, in dem der Kieferknochen durchstoßen wird, ein kritischer Moment, der vom Arzt eine große Erfahrung und ein besonderes Geschick erfordert, wobei trotz aller Umsicht ein Restrisiko besteht, die Kieferhöhlenschleimhaut beim Durchstoßen der Knochenplatte dennoch zu beschädigen. Um den Sinuslift-Eingriff sicherer zu machen, sind also Hilfsmittel wünschenswert, die dieses durchdringende Verlängern der Kieferknochenbohrung erleichtern, und dabei das Risiko, die zarte Sinushaut hinter dem Kieferknochen zu beschädigen, verringern.

Ein solches vorteilhaftes Hilfsmittel ist aus der WO 2010/048648 A1 bekannt, in der eine Vorrichtung beschrieben ist, die einen Rohrkörper mit einer distalen Arbeitsöffnung und einem der Arbeitsöffnung gegenüberliegenden Eingang aufweist, der mit einem von einem Schaft eines Arbeitswerkzeugs, z.B. eines Fräsers, durchsetzten Dichtungselement verschlossen ist. Am Rohrkörper ist ein Anschluss zum Aufbringen eines Innendruckes angeordnet. Der Rohrkörper wird in eine zuvor in den Kieferknochen eingebrachte Sackbohrung dichtend eingesetzt, wobei die distale Arbeitsöffnung am Ende der Sackbohrung ansteht, wodurch der Innenraum weitgehend dicht abgeschlossen ist. Das im Innenraum des Rohrkörpers befindliche Arbeitsmedium, vorzugsweise NaCI-Lösung, kann nun über den Anschluss unter Druck gesetzt werden, beispielsweise mittels einer mit dem Anschluss verbundenen Spritze. Mit dem Arbeitswerkzeug, das von außen gesteuert werden kann, wird nun die zwischen Sackbohrung und Kieferhöhle verbleibende Knochenscheibe im Bereich der Arbeitsöffnung abgefräst. In dem Moment, in dem der Kopf des Arbeitswerkzeugs den Knochen durchdringt und in den Bereich unterhalb der Sinushaut eindringt, bewirkt der Überdruck im Innenraum des Rohrkörpers, dass das Arbeitsmedium durch die freie Öffnung dringt und die dahinterliegende Sinushaut vom Knochen weg drückt und somit aus dem Arbeitsbereich des Arbeitswerkzeugs und dem Gefahrenbereich bringt. Das Ausströmen des Druckmediums bewirkt einen Druckabfall, der das Durchdringen des Knochens anzeigt und auch ein übermäßiges Aufblähen der Sinushaut verhindert.

Eine derartige Vorrichtung ist betriebssicher und lassen sich mit einer derartigen Vorrichtung chirurgisch ausgezeichnete Resultate mit minimalem Risiko für die Sinushaut erreichen. Ein gewisser Nachteil besteht allerdings darin, dass die Reinigung der Vorrichtung relativ aufwendig und schwierig ist. Vor allem ist es sehr schwierig, den dünnen Anschlusskanal, über den das Druckmedium in den Rohrkörper bzw. die Druckkammer zugeführt wird, so gründlich zu reinigen, dass keine Kontaminationen zurückbleiben. Grundsätzlich erzeugt jede Wiederverwendung von Medizinalprodukten bzw. Produkten im chirurgischen Bereich gewisse Infektionsrisiken beim Patienten und kann jede Kontamination zu gesundheitlichen Schäden, Erkrankungen oder sogar zum Tod des Patienten führen. Zwar kann man durch gewissenhaftes Resterilisieren der Vorrichtung einen Großteil der Keime abtöten, doch ist eine solche Resterilisierung zeitaufwendig, materialbeanspruchend und kostenintensiv und verbleibt trotzdem immer ein gewisses Restrisiko einer mikrobiellen Kontamination.

Es ist somit Aufgabe der vorliegenden Erfindung, die obengenannte Vorrichtung unter Beibehaltung ihrer vorteilhaften Funktionalität dahingehend weiterzuentwickeln, dass ihre Kontaminationssicherheit erhöht und das Infektionsrisiko für den Patienten gesenkt wird.

Diese Aufgabe wird mit der Schaffung eines gemäß den kennzeichnenden Merkmalen des Anspruchs 1 besonders ausgestalteten Führungselements gelöst.

Dieses erfindungsgemäße Führungselement kann als kostengünstiger austauschbarer Bauteil an der chirurgischen Vorrichtung angeordnet werden und ist als Verbrauchsmaterial für den Einmalgebrauch einsetzbar.

Das Führungselement ist speziell dazu geeignet, geformt und ausgelegt, um an einer Vorrichtung zur durchdringenden Verlängerung einer in hartes Gewebe, insbesondere den Kieferknochen, eingebrachten Sackbohrung angeordnet zu werden. Eine solche Vorrichtung umfasst einen, eine innere Druckkammer ausbildenden, Hohlkörper mit einer distalen Arbeitsöffnung und einer der Arbeitsöffnung gegenüberliegenden Eingangsöffnung. Diese Vorrichtung entspricht im wesentlichen auch der aus der WO 2010/048648 A1 bekannten Vorrichtung.

Das erfindungsgemäße Führungselement ist mit Passsitz formschlüssig in die Eingangsöffnung der Vorrichtung einsetzbar, wodurch diese durch das Einsetzen des Führungselementes dicht verschließbar ist. Das Führungselement weist außerdem eine durchgehende Ausnehmung auf, durch die ein Schaft eines Arbeitswerkzeuges, z.B. eines Fräsers, durchführbar ist und in den darunter liegenden Hohlkörper der Vorrichtung einführbar ist. Außerdem ist im Führungselement ein Anschluss für die Zuführung eines Arbeitsmediums zum Aufbringen des erforderlichen Innendruckes in der Druckkammer vorgesehen. Dieser Druck dient zum Wegdrücken der Sinusmembran nach dem Durchbrechen des Knochens.

Das erfindungsgemäße Führungselement gewährleistet damit zunächst, dass bei korrekter Applikation eine Druckkammer überhaupt ausgebildet bzw. ein ausreichender Druck aufgebaut werden kann. Außerdem erfolgt die Zufuhr des Druckmediums ebenfalls ausschließlich über bzw. durch das Führungselement hindurch, im Unterschied zur Vorrichtung der WO 2010/048648, bei der das Druckmedium über einen direkt in die Druckkammer einmündenden fixen seitlichen, schwer zu reinigenden Anschlussstutzen erfolgt. Neben diesen beiden Funktionen gewährleistet das Führungselement jedoch auch gleichzeitig, dass der Schaft des Arbeitswerkzeuges dichtend in die Sackbohrung eingeführt und gleichzeitig auch bewegt werden kann, um die verbleibende Bodenplatte zu entfernen.

Ein wesentlicher Vorteil ist, dass das Führungselement ausgetauscht werden und nach dem bestimmungsgemäßen Gebrauch abgenommen und entsorgt werden kann, wodurch die Sicherheit des chirurgischen Verfahrens steigt und das Infektionsrisiko für den Patienten stark reduziert wird.

Das Führungselement kann damit als separater, aseptisch verpackter Bauteil, ähnlich wie eine Spritze oder eine Injektionsnadel, hergestellt, vertrieben und gelagert werden und wird bei Bedarf unmittelbar vor der Operation aus der Verpackung genommen und mit dem chirurgischen Instrument verbunden und nach der Verwendung entsorgt. Eine solche Entsorgung wäre bei der Vorrichtung der WO 2010/048648 nicht sinnvoll, da es sich hierbei um einen teuren Präzisionsbauteil aus Edelstahl handelt. Auf diese Weise ist auch gewährleistet, dass ein allfälliges Ablaufdatum eingehalten bzw. nicht überschritten wird und die Betriebssicherheit gewährleistet ist.

In den Merkmalen der abhängigen Ansprüche sind weitere vorteilhafte Ausgestaltungen des erfindungsgemäßen Führungselementes beschrieben:
Besonders vorteilhaft ist es, wenn das Führungselement einen druckdichten und im wesentlichen fluiddichten Verschluss der Eingangsöffnung bewirkt. Zwar sind gewisse Undichtheiten oder auch ein Austreten von Druckmedium im geringen Umfang tolerierbar, doch lässt sich der erforderliche Druckaufbau in der Druckkammer und die Aufrechterhaltung dieses Druckes wesentlich besser bewerkstelligen, wenn das Führungselement die Eingangsöffnung möglicht dicht verschließt. Auch kann dadurch ein Druckabfall, der beim Durchbruch des Knochens auftritt, besser, schneller und sicherer erfasst werden und kann die Fräse rechtzeitig gestoppt werden.

Ebenso ist es äußerst vorteilhaft, wenn die Ausnehmung des Führungselements eine druckdichte und im wesentlichen fluiddichte Lagerung und Führung des Schaftes bewirkt, ebenfalls um den Innendruck in der Druckkammer von in der Regel etwa 2 bis 2,5 Bar überhaupt zu erreichen und diesen dann konstant aufrecht zu erhalten. Gleichzeitig muss die Ausnehmung jedoch so ausgestaltet sein, dass zumindest eine Vorschub-, Antriebs- und/oder Steuerbewegung des Schafts gewährleistet ist, beispielsweise eine Rotation, eine kreisende Bewegung und/oder ein axialer Vorschub des Schaftes beim Entfernen der Bodenplatte der Sackbohrung, ohne dass die abdichtenden Eigenschaften wesentlich beeinträchtigt werden.

In diesem Zusammenhang ist es besonders vorteilhaft, wenn im Inneren der Ausnehmung ein Dichtungselement, beispielsweise eine O-Dichtung, zur weiteren Abdichtung des Schafts angeordnet ist. Dadurch wird eine zusätzliche Dichtheit des Systems gewährleistet. Bei Ausführungsformen, bei denen ein herkömmlicher rotierend angetriebener Fräser verwendet wird, erfüllt das Dichtungselement die Funktion einer Wellendichtung und muss den rotierenden Schaft abdichten, ohne dessen Bewegungsfreiheit zu stark einzuschränken. Dies stellt verhältnismäßig hohe Anforderungen an die Qualität des Dichtungselements und führt zu einem raschen Verschleiß desselben.

Wenn das Führungselement einstückig bzw. einteilig, insbesondere als sterilisierbarer Einweg-Kunststoff-Spritzgußteil, ausgebildet ist, wird dadurch der Vorteil einer einfachen und preiswerten Herstellung erreicht sowie ein garantiert kontaminationsfreies Arbeiten gewährleistet.

Eine weitere vorteilhafte konstruktive Ausgestaltung des Führungselementes wird dadurch erreicht, indem die Ausnehmung, vorzugsweise mittig, in einem scheibenförmigen Kopfbereich des Führungselements angeordnet ist und der Anschluss als Stutzen zum Anschließen einer Schlauchleitung ausgebildet ist und sich, vorzugsweise radial, in der Ebene des scheibenförmigen Bereichs nach außen erstreckt.

Um eine sichere Zufuhr des Arbeits- bzw. Druckmediums zu erreichen, kann vorgesehen sein, dass im Führungselement ein innerer, allseitig geschlossener Kanal ausgebildet ist, der den Anschluss mit einer Austrittsöffnung verbindet, die in die Eingangsöffnung einmündet.

In diesem Zusammenhang ist vorteilhafterweise vorgesehen, dass der Kanal von der Ausnehmung durchwegs getrennt verläuft.

Besonders vorteilhaft ist es dabei, wenn der Kanal in einem ersten, dem Anschluss nahen, Teilabschnitt senkrecht zur zentralen Längsachse der Ausnehmung und in einem zweiten anschließenden Teilabschnitt im wesentlichen parallel zur zentralen Längsachse der Ausnehmung verläuft. Der Kanal kann auf diese Weise sehr einfach beispielsweise auch durch nachträgliche Bohrungen im Führungselement eingearbeitet werden.

Um ein dichtes, aber dennoch drehbares Einsetzen des Führungselementes in die Eingangsöffnung zu gewährleisten, ist gemäß einer weiteren vorteilhaften Ausgestaltung vorgesehen, dass auf der der Eingangsöffnung zugewendeten Seite des Führungselements ein, vorzugsweise im wesentlichen zylindrischer, Vorsprung ausgebildet ist, der vorzugsweise von einem Dichtungselement, beispielsweise einem O-Ring, umgeben ist.

Eine weitere vorteilhafte Ausgestaltung sieht vor, dass auf der der Eingangsöffnung zugewendeten Seite des Führungselements eine unten offene, sich verjüngende, kegelstumpfmantelförmige Hülse ausgebildet ist, die zentral von der Ausnehmung durchsetzt ist. Dadurch wird die Ausnehmung verlängert und die Dichtungswirkung sowie die Führung des Schaftes verbessert.

Gemäß einer besonders vorteilhaften Ausführungsform liegt die Hülse etwas enger und dichtender am Schaft an als der Rest der Ausnehmung. Die Hülse, insbesondere deren vorderster distaler Abschnitt, trägt damit am meisten zur Abdichtung bei und umgreift den Schaft besonders eng und dicht. Die anderen Abschnitte der Ausnehmung dichten zwar über ihre gesamte Länge ebenfalls gut ab, doch verbleibt hier ein gewisses geringes Spiel zwischen dem Schaft und der Innenfläche der Ausnehmung. Der Schaft kann sich dadurch in der Ausnehmung radial etwas bewegen, vorteilhaft unterstützt durch flexible Materialeigenschaften des Führungselements, die gewisse Verwindungen zulassen. Die Hülse ist bedingt durch ihre geringere Materialstärke ohnehin etwas flexibler und lässt taumelnde Bewegungen des Schafts zu. Die Hülse verbessert damit sowohl die Dichtheit als auch die Bewegbarkeit des Fräsers.

Konstruktiv ist es hierbei vorteilhaft, wenn die Hülse auf dem zylindrischen Vorsprung ausgebildet ist und der größte Durchmesser der Hülse vorzugsweise kleiner ist als der Durchmesser des Vorsprungs.

Da gemäß einer vorteilhaften Ausgestaltung das Führungselement mit einer Drehverschwenkung im chirurgischen Instrument eingesetzt wird, ist es vorteilhaft, wenn die der Eingangsöffnung zugewendete Unterfläche des Führungselements glatt und planeben ausgebildet ist, wodurch ein leichtes Verschwenken des Führungselementes ermöglicht wird.

Dabei ist es vorteilhaft, wenn ein seitlich vorstehendes Rastelement vorgesehen ist, das in eine korrespondierende Rastausnehmung der Vorrichtung reversibel einklickbar ist, wodurch eine sichere Betriebsstellung gewährleistet, aber auch ein leichtes Entfernen möglich ist.

Die Erfindung betrifft in einem weiteren Aspekt eine Vorrichtung bzw. ein chirurgisches System zur durchdringenden Verlängerung einer in hartes Gewebe, insbesondere in den Kieferknochen, eingebrachten Sackbohrung. Diese Vorrichtung umfasst ein Arbeitswerkzeug, z.B. einen Fräser, das erfindungsgemäße Führungselement sowie einen, eine Druckkammer ausbildenden, Hohlkörper. Dieser Hohlkörper hat einen, vorzugsweise zylindrischen, inneren Hohlraum mit einer distalen Arbeitsöffnung und einer der Arbeitsöffnung gegenüberliegenden Eingangsöffnung. Die Eingangsöffnung ist mit dem Führungselement verschlossen, weiters ist ein Schaft des Arbeitswerkzeugs durch die Ausnehmung durchgeführt bzw. durchführbar und in den Hohlraum des Hohlkörpers einführbar. Außerdem ist am Anschluss des Führungselements eine Schlauchleitung für ein fluides Arbeitsmedium zum Aufbringen eines Innendruckes in der Druckkammer anschließbar.

Mit einer derartigen Vorrichtung werden die oben erwähnten Vorteile erreicht und ist ein sicheres und rechtzeitiges Entfernen der Sinusmembran aus dem Gefahrenbereich, nämlich dem Arbeitsbereich des Arbeitswerkzeuges gewährleistet. Gleichzeitig wird das Kontaminationsrisiko erheblich verringert, da das Führungselement ausgetauscht werden kann.

Gemäß eine besonders vorteilhaften Ausführungsform ist der Bohrkopf der Fräse wesentlich kleiner als die Sackbohrung und besitzt eine kleinflächige, gegebenenfalls mit starker Krümmung gerundete, Spitze. Dadurch wird ein punktförmiges Durchstoßen der Knochenplatte gewährleistet, wodurch die Gefahr für die Sinushaut verringert wird. Auf diese Weise kann das Fluid bereits durch die kleine Erstöffnung hindurch strömen, noch bevor der Bohrkopf mit seinem gesamten Durchmesser durchbricht und mit der Sinushaut in Kontakt kommen könnte. Ein großflächiger Erstdurchbruch der Knochenplatte, wie dies mit flachen Bohrköpfen der Fall wäre, ist nicht vorteilhaft.

Vorteilhafte Ausgestaltungen dieser Vorrichtung sehen beispielsweise vor, dass das Führungselement die Eingangsöffnung und damit die Druckkammer druckdicht und im wesentlichen fluiddicht verschließt und im Inneren der Druckkammer vorzugsweise ein konstanter Druck von mindestens 2 bar erreichbar ist.

Weiters ist es vorteilhaft, dass der Schaft in der Ausnehmung druckdicht und im wesentlichen fluiddicht gelagert und geführt ist und im Inneren der Druckkammer vorzugsweise ein konstanter Druck von mindestens 2 bar erreichbar ist, dennoch aber gleichzeitig zumindest eine Vorschub-, Antriebs- und/oder Steuerbewegung des Schafts, beispielsweise eine Rotation, eine kreisende Bewegung und/oder ein axialer Vorschub des Schafts, gewährleistet ist. Wie einleitend erwähnt, ist dadurch der notwendige Druck zum Wegdrücken der Sinushaut, bei gleichzeitiger Beibehaltung der Beweglichkeit des Arbeitswerkzeuges, erreichbar und aufrecht erhaltbar.

Um eine leichte Entfernbarkeit des Führungselements zu gewährleisten und zu verhindern, dass das Führungselement aus Bequemlichkeitsgründen mehrfach verwendet wird, ist es vorteilhaft, wenn das Führungselement reversibel und zerstörungsfrei am Hohlkörper befestigbar und von diesem leicht und unkompliziert entfernbar ist.

Einen sehr einfache Handhabung kann man dadurch erzielen, dass am Hohlkörper ein Griffelement angeordnet ist. Damit kann der Chirurg bzw. Zahnarzt den Hohlkörper sicher in der Sackbohrung platzieren und wieder entfernen.

Eine konstruktiv vorteilhafte und leicht anzuwendende Ausgestaltung wird dadurch erreicht, dass das Führungselement in einem vertieften Bereich am distalen Ende des Griffelements angeordnet ist.

Wenn die Schlauchleitung in einer, gegebenenfalls mit Fixierelementen ausgestatteten, im Griffelement ausgebildeten, Nut verläuft, ist gewährleistet, dass die Schlauchleitung sicher und platzsparend gelagert ist und ein Abknicken oder ein unbeabsichtigtes Abreißen vom Anschluss nicht möglich ist.

Um eine stabile Betriebsstellung zu erreichen, kann gemäß einer vorteilhaften Weiterentwicklung vorgesehen sein, dass eine Rastausnehmung, vorzugsweise in der Randfläche des vertieften Bereichs, ausgebildet ist, in die das korrespondierende Rastelement des Führungselements einklickbar und das Führungselement in dieser Lage fixierbar ist, wobei die Verschränkung insbesondere durch eine Drehverschwenkung des Führungselements um etwa 30° bis 50°, mit der Ausnehmung als Drehachse, erfolgt.

In diesem Zusammenhang ist es vorteilhaft, insbesondere um eine leichte Drehbarkeit zu erreichen, wenn das Führungselement mit dem zylindrischen Vorsprung fluiddicht in die Eingangsöffnung, insbesondere bis zu einem Anschlag, einsetzbar ist.

Damit die gleichzeitige Zufuhr des Druckmediums sowie die Durchführung des Schaftes durch die Ausnehmung durch das Führungselement möglich ist, ist es vorteilhaft, wenn die Eingangsöffnung zumindest abschnittsweise in Form eines sich von einem breiteren Anfangsbereich konisch verjüngenden trichterförmigen Hohlbereichs ausgebildet ist. Hierbei ist es vorteilhaft, wenn die Außenwandung der Hülse von der trichterförmigen Innenwandung der Eingangsöffnung beabstandet ist.

Zum abdichtenden Einsetzen in die Knochenbohrung kann der in die Sackbohrung einzubringenden oberste Teilbereich des Hohlkörpers konisch ausgebildet sein. Der konische Bereich kann mit manueller Kraft in die Sackbohrung gepresst werden. Dies ist insbesondere dann vorteilhaft, wenn eine Griffverlängerung vorgesehen ist, die ein Drehen des in die Bohrung eingesetzten Instruments im Mundbereich behindert. Das Drehen des Instruments kann dazu dienen, das Instrument in die Bohrung einzuschrauben. Zu diesem Zweck kann in einer weiteren Ausführungsform am Hohlkörper in dem in die Sackbohrung einzubringenden Bereich ein selbstschneidendes Außengewinde vorgesehen sein. Dieses sorgt für einen sicheren Halt des Hohlkörpers in der Bohrung und verbessert die Abdichtung gegenüber der Innenwandung der Bohrung.

Um diese Abdichtung weiter zu verbessern, kann in vorteilhafter Weise an der Außenseite des Hohlkörpers ein entlang des Hohlkörpers verstellbarer und an diesem lösbar festlegbarer Flansch vorgesehen sein, der einen konischen, zwischen Hohlkörper und Sackbohrungswandung reichenden Dichtungsansatz aufweist. Dieser Flansch wird mit dem Konus gegen die Mundschleimhaut gepresst und dann, z.B. mittels einer Inbusschraube, fixiert, wodurch auch dieses Ende der Druckkammer druckdicht verschlossen ist.

Um einen langsamen Vorschub des Fräskopfes zu erreichen und ein stoßartiges Vordringen möglichst zu verhindern, kann vorgesehen sein, dass zwischen dem Führungselement und dem Arbeitswerkzeug ein elastischer Faltenbalg angeordnet ist.

Gemäß einer weiteren vorteilhaften Ausgestaltung ist das Arbeitswerkzeug ein rotierend angetriebenes Arbeitswerkzeug, z.B. ein Fräser, oder ein rotationsfreies Arbeitswerkzeug, beispielsweise ein Ultraschall-Osteotom oder ein Laserschneidegerät. Derartige Arbeitswerkzeuge sind ausführlich in der WO 2010/048648 A1 beschrieben und werden in den Inhalt der vorliegenden Anmeldung aufgenommen. Rotationsfrei bedeutet im Zusammenhang mit dieser Erfindung, dass das Arbeitswerkzeug und das Dichtungselement im wesentlichen ohne auftretende Relativgeschwindigkeiten aneinander anliegen, da das Arbeitswerkzeug nicht um seine Hauptachse rotiert. Dadurch entfällt das Erfordernis, einen rotierenden Schaft mittels einer Rotations-Wellendichtung abzudichten, was die Anforderungen für das Dichtungselement verringert.

Das Arbeitswerkzeug kann ein piezoelektrisches chirurgisches Instrument, vorzugsweise ein im Mikrometerbereich, insbesondere in einem Bereich zwischen 20 und 200 Mikrometer, arbeitendes Ultraschall-Osteotom, sein. Alternativ kann das Arbeitswerkzeug ein Laserschneidegerät sein, das einen vorzugsweise gepulsten CO2-Laser oder Festkörperlaser aufweist, wobei die Wellenlänge des Lasers vorzugsweise auf die Absorptionscharakteristik des Knochengewebes abgestimmt ist. Zusätzlich kann der Laser eine verstellbare Fokussierung aufweisen und mit einem Endoskop gekoppelt sein.

In einer weiteren vorteilhaften Ausgestaltung der Erfindung kann die Fräseinrichtung über den Anschluss mit einer manuellen oder automatischen Drucksteuereinheit verbunden sein. Diese ermöglicht ein exaktes Steuern und Kontrollieren des Innendrucks, wobei das Durchstoßen des Knochens sofort anhand des Druckabfalls erkennbar ist und gegebenenfalls die Fräse abgeschaltet werden kann.

Wenn die Vorrichtung eine Einrichtung zur Erzeugung bzw. Übertragung mechanischer oder elektromagnetischer Schwingungen aufweist, so können zusätzlich Schwingungen, vorzugsweise drucklose Ultraschallschwingungen, in das System bzw. das Arbeitsmedium eingebracht werden, um ein Ablösen der Sinusmembran zu beschleunigen.

Bei einer weiteren vorteilhaften Ausgestaltung ist vorgesehen, dass sich der Durchmesser des Schafts zum Bohrkopf hin mit zumindest einer Stufe verringert. Dabei weist der Teilabschnitt des Schafts, der im Bereich der Druckkammer liegt, einen geringeren Durchmesser auf als der Teilabschnitt des Schafts, der dichtend im Bereich der Ausnehmung liegt. Auf diese Weise ist der Schaft dicht in der Ausnehmung gelagert, es verbleibt jedoch eine räumlich ausreichend große Druckkammer und der Schaft ist zudem ausreichend bewegbar, um die verbleibende Knochenplatte möglichst vollständig abzutragen.

Die Erfindung betrifft außerdem ein Set umfassend das erfindungsgemäße Führungselement sowie eine Schlauchleitung zum Anschließen an das Führungselement und gegebenenfalls eine Spritze zum Aufbringen des erforderlichen Innendruckes, wobei das Führungselement und die Schlauchleitung sowie die Spritze sterilisiert und in einem geschlossenen Behältnis, vorzugsweise einem Kunststoffbeutel, aseptisch verpackt vorliegen. Dies erleichtert die Lagerung und Handhabung und sichert ein kontaminationsfreies Arbeiten.

Weitere Vorteile und Ausgestaltungen der Erfindung ergeben sich aus der Beschreibung und den beiliegenden Zeichnungen.

Die Erfindung ist nunmehr anhand eines besonders vorteilhaften, aber nicht einschränkend zu verstehenden, Ausführungsbeispiels in den Zeichnungen schematisch dargestellt und wird im Folgenden unter Bezugnahme auf die Zeichnungen beispielsweise beschrieben:
Fig. 1 zeigt das erfindungsgemäße Führungselement in einer perspektivischen Ansicht von schräg oben.
Fig. 2 zeigt das Führungselement gemäß Fig. 1 in einer Ansicht von schräg unten.
Fig. 3 zeigt die gesamte Vorrichtung mit dem erfindungsgemäßen Führungselement, dem Hohlkörper sowie einem Arbeitswerkzeug.
Fig. 4 bis 7 zeigen schrittweise die Vorbereitung und Zusammenstellung der Vorrichtung:

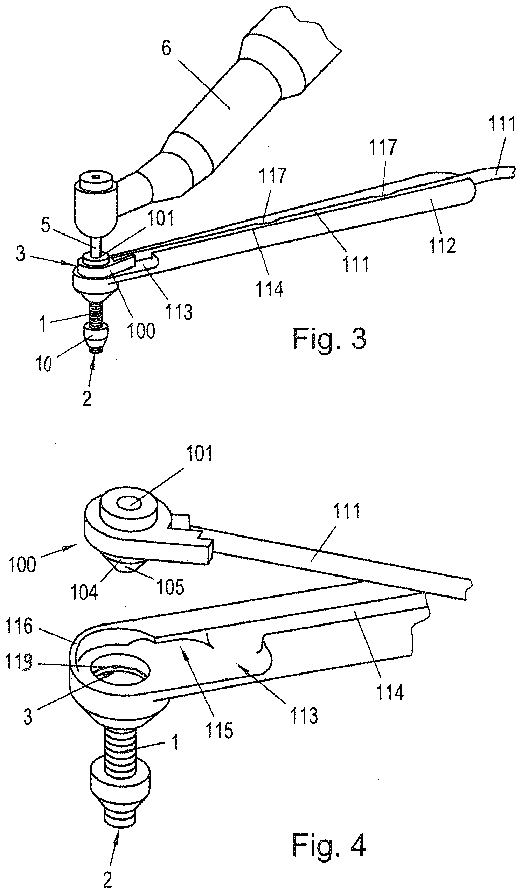

   Fig. 4 zeigt das Einsetzen des Führungselements in den Hohlkörper.

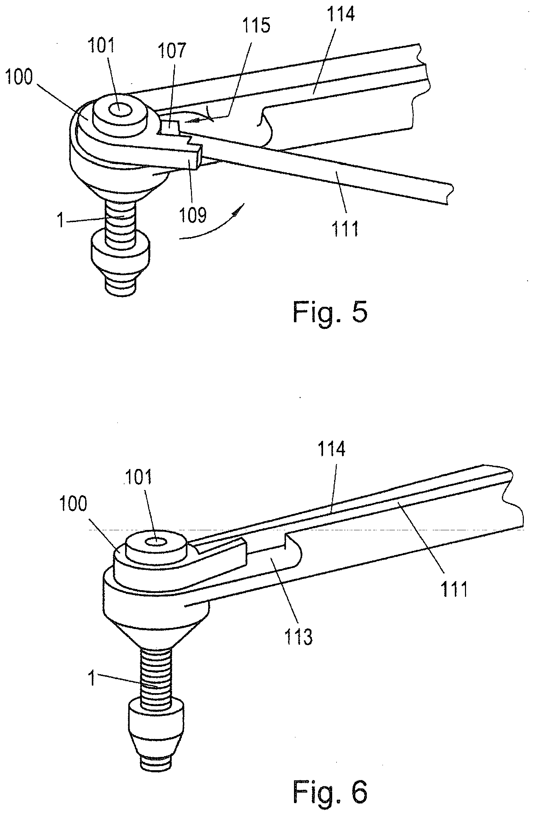

   Fig. 5 zeigt das Verschwenken des eingesetzten Führungselementes.
   Fig. 6 zeigt die Vorrichtung nach dem Einrasten des Führungselements.

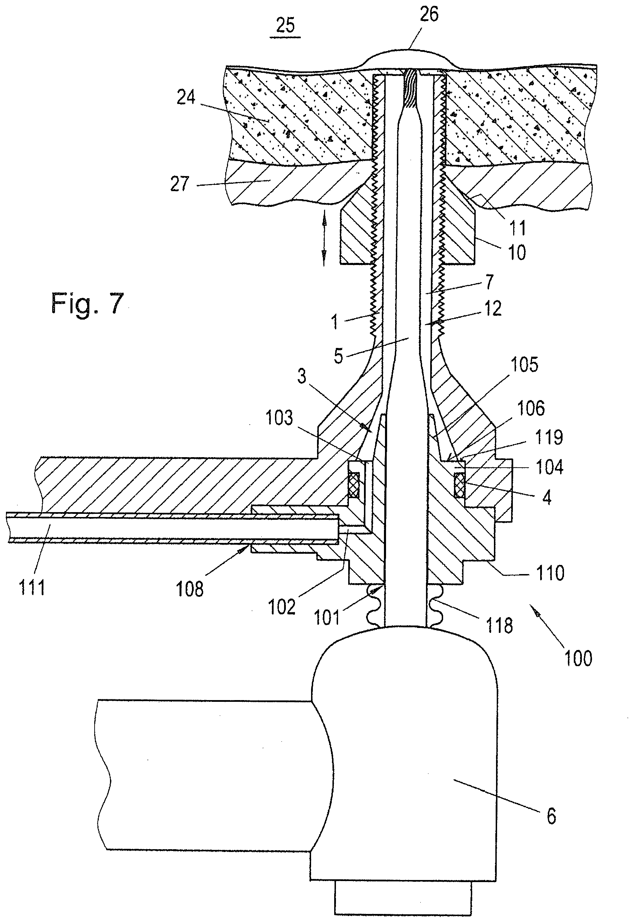

   Fig. 7 zeigt einen Querschnitt durch die Vorrichtung mit eingesetztem Schaft des Arbeitswerkzeugs im Zeitpunkt des Durchbrechens des Arbeitswerkzeugs durch die Knochenplatte.

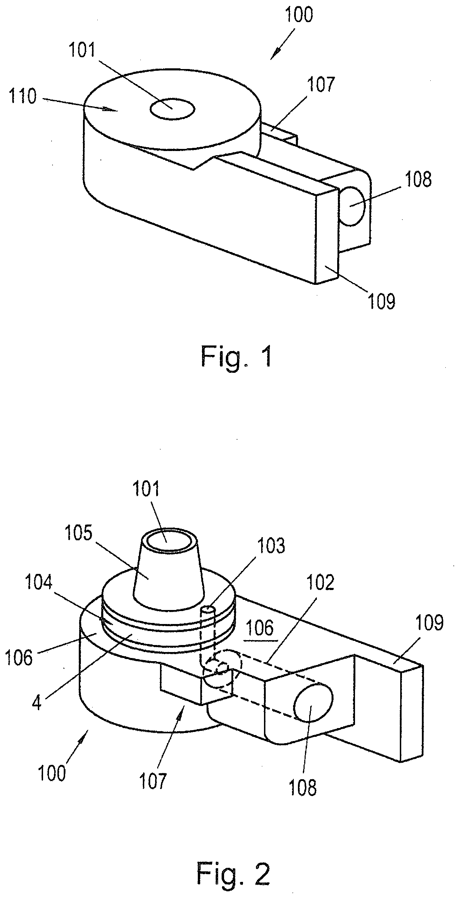

In Fig. 1 ist ein besonders vorteilhaftes Ausführungsbeispiel des erfindungsgemäßen Führungselements 100 in einer Schrägansicht von oben dargestellt. Das hier gezeigte Führungselement 100 besteht aus einem sterilisierbaren Polymer, wie dies im Bereich der Medizintechnik bekannt ist. Das Führungselement 100 ist einstückig bzw. einteilig und wird mittels Spritzgussverfahren hergestellt. Nachträglich können jedoch Dichtungen oder Bohrungen vorgenommen werden.

In einem scheibenförmigen Kopfbereich 110 des Führungselements 100 ist zentral mittig eine zylinderförmige Ausnehmung 101 ausgebildet, die das Führungselement 100 vollständig durchsetzt. In diese Ausnehmung 101 wird im Betrieb der Schaft 5 eines Arbeitswerkzeuges 6, z.B. eines Fräsers, durchgeführt. Der Durchmesser der Ausnehmung 101 ist etwas größer als der des Schaftes 5 des Arbeitswerkzeugs 6, sodass der Fräser 5, 6 im Inneren des Hohlkörpers 1 bewegbar bleibt und von Arbeitsmedium umspült werden kann.

An diesen Kopfbereich 110 schließt sich ein Anschluss 108 in Form eines Stutzens zum Befestigen einer in dieser Figur nicht dargestellten Schlauchleitung 111 an. Die Schlauchleitung 111 wird in diesen Stutzen eingeschoben. Der Anschluss 108 erstreckt sich radial vom Mittelpunkt der Ausnehmung 101 nach außen und die zentrale Längsachse des Anschlusses 108 liegt in der parallel zur Oberfläche ausgerichteten Ebene des scheibenförmigen Kopfbereichs 110.

Neben dem Anschluss 108 ist eine vorstehende Nase 109 ausgebildet, die sich tangential vom Kopfbereich 110 parallel zum Anschluss 108 erstreckt und den Anschluss 108 sowohl in ihrer Höhe als auch in der Längserstreckung überragt. Die Nase 109 dient einerseits zur Sicherung des Anschluss der Schlauchleitung 111 sowie zur besseren Angreifbarkeit bei der Verschwenkung.

Zwischen dem Kopfbereich 110 und dem Anschluss 108 ist ein leicht nach unten versetztes vorstehendes Rastelement 107 angeformt, mit dem das Führungselement 100 reversibel an der Vorrichtung befestigt werden kann.

Wie in Fig. 3 bis 7 ersichtlich, kann, insbesondere zur Verbesserung der Führung und der Dichtheit, auf der Oberseite des Führungselements 100 eine um die Ausnehmung 101 herum angeordnete zylindrische Erhöhung ausgebildet sein.

In Fig. 2 wird das Führungselement 100 von der gegenüberliegenden Seite von schräg unten gezeigt. Die im Betrieb dem Knochen 24 zugewendete Unterfläche 106 des Führungselements 100 ist glatt und planeben ausgebildet, wodurch eine gute Gleitfähigkeit und Drehverschwenkbarkeit gewährleistet ist. Im Kopfbereich 110 ist ein zylindrischer Vorsprung 104 ausgebildet, der von einem in einer Nut teilweise versenkten Dichtungselement 4 in Form eines O-Rings umgeben ist. Damit wird das Führungselement 100 in die Vorrichtung passsitzig eingesetzt, wie in den kommenden Figuren beschrieben.

Auf dem Vorsprung 104 ist eine kegelstumpfmantelförmige Hülse 105 angeformt, die sich nach oben hin verjüngt. Der größte Durchmesser an der Basis der Hülse 105 ist um etwa 30 bis 40 % kleiner als der Durchmesser des zylindrischen Vorsprungs 104.

Sowohl der zylindrische Vorsprung 104 als auch die Hülse 105 sind ebenso wie der Kopfbereich 110 konzentrisch um die zentrale Längsachse der Ausnehmung 101 angeordnet und werden durch die Ausnehmung 101 mittig durchsetzt.

Ausgehend vom Anschluss 108 erstreckt sich ein vollständig im Inneren des Führungselementes 101 liegender allseitig geschlossener Kanal 102, der den Anschluss 108 mit einer Austrittsöffnung 103 verbindet. Diese Austrittsöffnung 103 mündet an der Unterfläche des zylindrischen Vorsprungs 104 neben der Hülse 105 und gewährleistet die Einbringung des fluiden Arbeits- bzw. Druckmediums, meist physiologische Kochsalzlösung, in die Eingangsöffnung 3 der Vorrichtung. Der Kanal 102 ist von der Ausnehmung 101 durchwegs getrennt. Der Kanal 102 weist dabei einen dem Anschluss 108 nahen geraden ersten Teilabschnitt mit anfänglich größerem Durchmesser auf, der im wesentlichen senkrecht und radial zur zentralen Längsachse der Ausnehmung 101 ausgerichtet ist. Der Kanal 102 verkleinert dann seinen Durchmesser und biegt im rechten Winkel ab. Dieser anschließende zweite Teilabschnitt verläuft im wesentlichen parallel zur zentralen Längsachse der Ausnehmung 101. Auf diese Weise kann der Kanal 102 konstruktiv einfach, beispielsweise durch zwei nachträgliche Bohrungen, im einstückigen Führungselement 100 ausgebildet werden.

In Fig. 3 ist die erfindungsgemäße Vorrichtung in perspektivischer Ansicht dargestellt. Diese exemplarische und nicht einschränkend zu verstehende Vorrichtung zur durchdringenden Verlängerung einer Sackbohrung im Kieferknochen 24 umfasst ein Arbeitswerkzeug 6 in Form eines Fräsers 6 sowie das oben beschriebene Führungselement 100, das fix mit einem Hohlkörper 1 verbunden ist.

Der Hohlkörper 1 ist an einem Griffelement 112 angeordnet, welches eine Form eines abgeflachten Stäbchens aufweist. Das Führungselement 100 ist dabei in einem vertieften Bereich 113 am vorderen Ende des Griffelements 112 angeordnet. Der Hohlkörper 1 besitzt einen im wesentlichen zylindrischen inneren Hohlraum 12, eine distale Arbeitsöffnung 2 sowie eine dieser Arbeitsöffnung 2 gegenüberliegende Eingangsöffnung 3. Durch diese Eingangsöffnung 3 wird sowohl das Druckmedium als auch der Schaft 5 des Arbeitswerkzeugs 6 in den Hohlraum 12 des Hohlkörpers 1 eingeführt. Der Hohlkörper 1 ist im wesentlichen so aufgebaut, wie der in der WO 2010/048648 A1 beschriebene Rohrkörper.

Um im Inneren des Hohlkörpers 1 eine Druckkammer 7 ausbilden zu können, ist es erforderlich, sowohl den Hohlraum 12 des Hohlkörpers 1 und den Schaft 5 des Fräsers 6 im Bereich der Eingangsöffnung 3 abzudichten. Durch diese Druckkammer 7 und den darin erzeugten Druck ist ein rechtzeitiges Wegdrücken der Sinusmembran 26 aus dem Gefahrenbereich beim Durchstoßen der Knochenplatte gewährleistet.

Die sich ausbildende Druckkammer 7 kann dabei entweder vollständig im Inneren des Hohlkörpers 1 liegen, wenn die Arbeitsöffnung 2 dichtend mit dem Boden der Sackbohrung abschließt. Die Druckkammer 7 kann sich aber auch bis in einen Bereich außerhalb des eigentlichen Hohlkörpers 1 erstrecken, der durch die Wandung der Sackbohrung und die konische Dichteinheit bzw. den Flansch 11 begrenzt ist. Im Gebrauch wirkt beides dichtend und ergänzen sich beide Dichtungen, um das System möglichst druck- und fluiddicht zu halten.

Die Eingangsöffnung 3 am anderen Ende der Druckkammer 7 ist durch das passsitzig und formschlüssig eingesetzte Führungselement 100 druck- und fluiddicht verschlossen. Außerdem ist der Schaft 5 bereits eingesetzt und durchsetzt die Ausnehmung 101, tritt allerdings durch die Arbeitsöffnung 2 noch nicht aus, ist somit noch nicht betriebsbereit. Am Anschluss 108 ist die Schlauchleitung 111 bereits angeschlossen und damit die Zuführung von Druckmedium ins Innere des Hohlkörpers 1 möglich. Die Schlauchleitung 111 verläuft in einer im Griffelement 112 ausgebildeten vertieften Nut 114. Diese Nut ist an zwei Stellen mäanderförmig gebogen, um den Schlauch an diesen Fixierelementen 117 festzulegen. Die Schlauchleitung 111 kann zu einer Spritze oder einer manuellen oder automatischen Drucksteuereinheit führen, mit der der Druck einbringbar und kontrollierbar ist.

In den Fig. 4 bis 7 wir.d schrittweise die Vorbereitung der Vorrichtung für den Eingriff dargestellt:
In Fig. 4 ist das Führungselement 100 zwar bereits mit der Schlauchleitung 111 verbunden, jedoch noch nicht am Hohlkörper 1 angesetzt. Im vorderen Teil des Griffelements 112 ist der vertiefte Bereich 113 erkennbar, in dem das Führungselement 100 angeordnet wird. Im vordersten distalen Bereich des vertieften Bereichs 113 ist die Eingangsöffnung 3 ausgebildet, die den Zugang zum inneren Hohlraum 12 des Hohlkörpers 1 definiert. Die Eingangsöffnung 3 ist zu Beginn sehr breit und in einem ersten Abschnitt zylindrisch ausgebildet bis hin zu einem Anschlag bzw. einer umlaufenden Anschlagsfläche 119 mit im Vergleich zur Ausnehmung 101 verringertem Durchmesser. In diesen zylindrischen Bereich der Eingangsöffnung 3 wird der zylindrische Vorsprung 104 des Führungselements 100 passsitzig eingesetzt, wobei die Unterfläche des zylindrischen Vorsprungs 104 dann auf dem Anschlag 119 aufliegt. Dadurch wird eine sichere Verdrehbarkeit bzw. Verschwenkbarkeit bei gleichzeitiger Führung und Dichtheit gewährleistet, wobei die zentrale Längsachse der Ausnehmung 101 die Drehachse bildet.

Die Eingangsöffnung 3 verjüngt sich dann konisch nach unten zu und bildet einen trichterförmigen Hohlbereich. Im Inneren des Hohlkörpers 1 verläuft der im wesentlichen zylindrische Hohlraum 12 bis hin zur Arbeitsöffnung 2.

In Fig. 5 ist das Führungselement 100 bereits mit dem zylindrischen Vorsprung 104 in die Eingangsöffnung 3 passsitzig und dicht eingesetzt. Das Führungselement 100 wird dann durch eine Drehung bzw. Verschwenkung um etwa 30° bis 40° in Pfeilrichtung verdreht, bis der Anschluss 108 im wesentlichen auf den Beginn der Nut 114 zeigt. Praktisch erfolgt die Verschwenkung durch Angreifen und Druckbeaufschlagung an der Nase 109. Das Rastelement 107 ist dabei form- und kraftschlüssig in die korrespondierende Rastausnehmung 115 im Griffelement 112, genauer in der vertikalen Randfläche des vertieften Bereichs 113 kraftschlüssig einklickbar, und das Führungselement 100 dadurch lagefixiert.

In Fig. 6 ist diese Verschränkung bereits erfolgt und auch die Schlauchleitung 111 bereits in der Nut 114 eingesetzt und fixiert. Nun ist das Gerät vorbereitet und erfolgt in der Praxis zumeist das dichtende Einsetzen des Hohlkörpers 1 in die bereits vorgebohrte Sackbohrung im Kieferknochen 24 des Patienten.

Fig. 7 zeigt einen Querschnitt durch die Vorrichtung mit eingesetztem Schaft 5 des Arbeitswerkzeugs 6 bei einem Sinuslift im Zeitpunkt des Durchbrechens des Arbeitswerkzeugs 6 durch die Knochenplatte des Kieferknochens 24 des Oberkiefers. Fig. 7 zeigt die Vorrichtung in ihrer in der Praxis räumlich korrekten Positionierung relativ zum Patienten.

Wie beim herkömmlichen crestalen Sinuslift wird in einem vorausgehenden Eingriff vom Kieferkamm her eine Sackbohrung in den Kieferknochen 24 eingebracht, wobei eine etwa 1 mm tiefe Knochenplatte zwischen dem Ende der Sackbohrung und der Kieferhöhle 25 verbleibt. Dies ist notwendig, um die in der Kieferhöhle 25 am Kieferknochen 24 anliegende Kieferhöhlenschleimhaut 26 nicht zu beschädigen.

In die vorbereitete Sackbohrung wird dann der Hohlkörper 1 dichtend eingesetzt, bis die Arbeitsöffnung 2 an der Knochenplatte ansteht. Der Hohlkörper 1 steht während der Behandlung still, ist lagefixiert und kann sogar in die Sackbohrung eingeschraubt werden.

Zur Verbesserung der abdichtenden Wirkung wird der Flansch 10 auf dem Rohrkörper 1 zum Kieferknochen 24 hin verschoben, sodass der auf dem Flansch 10 angeordnete konische Dichtungsansatz 11 am äußeren Rand der Sackbohrung fest gegen die Mundschleimhaut 27 gedrückt wird und die Sackbohrung dadurch abdichtet. Gegebenenfalls kann zusätzlich auch ein Kofferdam verwendet werden. Im Inneren der Druckkammer 7 kann sich dadurch in weiterer Folge ein hydrostatischer Druck von beispielsweise etwa 0,5 bis 3 bar ausbilden.

Das Arbeitsmedium in der Druckkammer 7 dient dabei gleichzeitig der Abfuhr der beim Fräsen erzeugten Wärme und wirkt für den rotierenden Schaft 5 als Schmiermittel. Geringe Mengen an Arbeitsmedium, die entlang des rotierenden Schafts 5 austreten können, stellen für die Funktionalität der Einrichtung kein Problem dar, da der Druck des Arbeitsmediums in der Druckkammer 7 über den Anschluss 108 aufrechterhalten werden kann. Gute Dichteigenschaften sind dennoch sehr vorteilhaft, da der Druckabfall im Moment des Durchdringens der Knochenscheibe bei einer dichten Druckkammer 7 besser erkennbar ist.

Dann wird zunächst der Schaft 5 des Arbeitswerkzeugs 6 durch die Ausnehmung 101 und die Eingangsöffnung 3 hindurch in das Innere des Hohlkörpers 1 eingeführt. Der Durchmesser des Schafts 5 ist stufenweise zur Spitze hin verjüngt ausgebildet. So verringert sich der Durchmesser des Schafts 5 im Bereich der Einmündung der Eingangsöffnung 3 um etwa ein Viertel. Dadurch verbleibt zwischen dem Schaft 5 und der Innenfläche des Hohlraums 12 genug Raum, um die Druckkammer 7 auszubilden sowie um dem Fräser 6 genug Bewegungsspielraum für taumelnde Bewegungen zu geben. Im endständigsten Bereich des Bohrkopfs des Fräsers 6 verringert sich der Durchmesser erneut um etwa ein Viertel. Die äußerste Spitze bzw. der Bohrkopf des Fräsers 6 bzw. des Schafts 5 ist zudem relativ stark gekrümmt bzw. im wesentlichen punktförmig ausgestaltet, um einen möglichst punktförmigen und kleinflächigen Durchtritt durch die Knochenplatte zu gewährleisten.

Der Schaft 5 durchsetzt dabei die Ausnehmung 101, die Eingangsöffnung 3 sowie den inneren Hohlraum 12 des Hohlkörpers 1 vollständig und tritt im Betrieb durch die Ausgangsöffnung 2 aus, damit die verbleibende Knochenplatte bearbeitet werden kann. Der distale Bereich des Schafts 5 mit geringerem Durchmesser lässt sich recht einfach durch die Ausnehmung 101 hindurchführen, der hintere Bereich mit größerem Durchmesser fügt sich dann passsitzig in die Ausnehmung 101 ein.

Der Schaft 5 liegt damit in diesem Bereich dichtend an der Innenfläche der Ausnehmung 101 an bzw. ist zusätzlich auch im Bereich des zylindrischen Vorsprungs 104 und der Hülse 105 gedichtet, geführt und gestützt. Im Inneren der Ausnehmung 101 könnte auch eine zusätzliche Dichtung, beispielsweise ein O-Ring vorgesehen sein, im vorliegenden Ausführungsbeispiel ist dies jedoch nicht der Fall.

Dennoch ist der Schaft 5 axial, das heißt gemäß dem Pfeil nach oben und unten, verschiebbar, was vor allem bei rotierenden Fräsern 6 erforderlich ist, um den notwendigen Vorschub für die Durchfräsung der Bodenplatte im Kieferknochen 24 zu erreichen. Außerdem ist es weiterhin möglich, beispielsweise bedingt durch die Flexibilität des Materials des Führungselements 100, den Schaft 5 in einer kreisenden bzw. taumelnden Bewegung zu bewegen, um die knöcherne Bodenplatte möglichst großflächig und vollständig abzutragen.

Verwendet man ein Arbeitswerkzeug mit einem um die eigene Achse rotierenden Schaft 5, so ist auch diese Rotationsbewegung möglich. Dennoch ist der Schaft 5 in der Ausnehmung 101, vor allem im Bereich der Hülse 105, fluiddicht und druckdicht gelagert und kann in der Druckkammer 7 ein ausreichender Druck aufgebaut werden, wenn der Hohlkörper 1 dicht in der Sackbohrung eingesetzt ist.

Über den Anschluss 108 ist nun das Arbeitsmedium, beispielsweise eine physiologische Kochsalzlösung durch den Kanal 102 und die Austrittsöffnung 103 in die Eingangsöffnung 3 einführbar. Da die Außenwandung der Hülse 105 von der Innenwandung der trichterförmigen Eingangsöffnung 3 beabstandet ist, fließt das Spülmedium außen der Hülse 105 entlang und entlang des Schaftes 5 in den inneren Hohlraum 12 bzw. die Druckkammer 7.

Der Fräser 6 in Fig. 7 wird dann im Betrieb immer weiter nach unten verschoben, damit der Fräskopf am unteren Ende durch die Arbeitsöffnung 2 austritt und die Bodenplatte berührt, wobei der Vorschub des Schafts 5 bzw. Fräsers 6 sehr langsam, beispielsweise etwa 1 mm/min, ist.

Sobald die Bodenplatte an einer Stelle minimal durchstoßen wird, was durch den punktförmigen Bohr- bzw. Fräskopf unterstützt wird, dringt das unter Druck stehende Arbeitsmedium sofort durch diese minimale Öffnung hindurch und wird die Sinusmembran 26 unmittelbar aus dem Gefahrenbereich vom Bohrkopf weggedrückt und vom Kieferknochen 24 etwas gelöst, noch bevor der Bohrkopf vollständig durch die Öffnung durchtreten kann. Dies entspricht gerade der in Fig. 7 dargestellten Situation.

Der Fräser 6 wird dann abgeschaltet und es erfolgt, wie aus der WO 2010/048648 A1 bekannt, ein weiteres Ablösen der Sinusmembran 26 um dem Raum für das Knochenersatzmaterial zu vergrößern, beispielsweise durch zusätzliches Zuführen von Druckmedium und gegebenenfalls Einbringen von Schwingungen.

Der Schaft 5 kann dabei zunächst als dichtendes Element in der Ausnehmung 101 verbleiben. Alternativ kann der Schaft 5 auch entfernt werden und die Ausnehmung 101 durch Einsetzen eines Stopfens dicht verschlossen werden. Dadurch kann weiter Druck ausgeübt werden bzw. der Druck konstant aufrechterhalten werden.

Gegebenenfalls können auch Schwingungen, beispielsweise Ultraschallschwingungen, ins Medium eingebracht werden, um ein weiteres Ablösen der Sinusmembran 26 zu erleichtern.

Zum Austauschen bzw. Entfernen des Führungselements 100 nach dem Eingriff wird das Führungselement 100 an der Nase 109 angegriffen und in die entgegengesetzte Pfeilrichtung in Fig. 5 drehverschwenkt. Im vordersten Bereich des Griffelements 112 ist ein kontinuierlich ansteigender äußerer Rand 116 ausgebildet, der mit der Unterfläche 106 in Wirkverbindung tritt, das Führungselement 100 beim Verschwenken nach oben zwingt und schlussendlich aus der Eingangsöffnung 3 abhebt.

Erfindungsgemäß ist weiters vorgesehen, dass das Führungselement 100, entweder alleine oder kombiniert mit der Schlauchleitung 111, als sterilisierter und aseptisch verpackter Einweggegenstand bzw. Medizinalprodukt vertrieben wird. Kurz vor Beginn des Eingriffs kann der Chirurg bzw. Zahnarzt die Verpackung aufreißen und das garantiert kontaminationsfreie Führungselement 100 sowie die Schlaucheinheit 111 entnehmen, am Hohlkörper 1 befestigen und das Arbeitswerkzeug entsprechend anordnen.

Für die Funktionalität der Vorrichtung und insbesondere die Anwendung am Patienten wird ergänzend auf die Ausführungen in der WO 2010/048648 A1 verwiesen.

## Patentansprüche

1. Führungselement (100) zum Anordnen an einer Vorrichtung zur durchdringenden Verlängerung einer in hartes Gewebe, insbesondere in den Kieferknochen (24), eingebrachten Sackbohrung, wobei diese Vorrichtung einen, eine Druckkammer (7) ausbildenden, Hohlkörper (1) mit einer distalen Arbeitsöffnung (2) und einer der Arbeitsöffnung (2) gegenüberliegenden Eingangsöffnung (3) umfasst, wobei das Führungselement (100) mit Passsitz in die Eingangsöffnung (3) einsetzbar ist und die Eingangsöffnung (3) durch das Führungselement (100) verschließbar ist, wobei das Führungselement (100) eine durchgehende Ausnehmung (101) aufweist, durch die ein Schaft (5) eines Arbeitswerkzeugs (6), z.B. eines Fräsers, durchführbar und in den Hohlkörper (1) einführbar ist und wobei das Führungselement (100) einen Anschluss (108) für ein Arbeitsmedium zum Aufbringen eines Innendruckes in der Druckkammer (7) bzw. im Hohlkörper (1) aufweist, **dadurch gekennzeichnet, dass** im Führungselement (100) ein innerer, allseitig geschlossener Kanal (102) ausgebildet ist, der den Anschluss (108) mit einer Austrittsöffnung (103) verbindet, die in die Eingangsöffnung (3) einmündet und dass der Kanal (102) von der Ausnehmung (101) durchwegs getrennt verläuft.

2. Führungselement nach Anspruch 1, **dadurch gekennzeichnet, dass** das Führungselement (100) einen druckdichten und im wesentlichen fluiddichten Verschluss der Eingangsöffnung (3) bewirkt,
und/oder dass die Ausnehmung (101) eine druckdichte und im wesentlichen fluiddichte Lagerung und Führung des Schafts (5) bewirkt und gleichzeitig zumindest eine Vorschub-, Antriebs- und/oder Steuerbewegung des Schafts (5), beispielsweise eine Rotation, eine kreisende bzw. taumelnde Bewegung und/oder einen axialen Vorschub des Schafts (5), gewährleistet.

3. Führungselement nach einem der Ansprüche 1 bis 2, **dadurch gekennzeichnet, dass** im Inneren der Ausnehmung (101) ein Dichtungselement, beispielsweise eine O-Dichtung, zur weiteren Abdichtung des Schafts (5) angeordnet ist.

4. Führungselement nach einem der Ansprüche 1 bis 2, **dadurch gekennzeichnet, dass** das Führungselement (100) einstückig bzw. einteilig, insbesondere als sterilisierbarer Einwegbauteil, vorzugsweise aus Kunststoff, ausgebildet ist.

5. Führungselement nach einem der Ansprüche 1 bis 4, **dadurch gekennzeichnet, dass** die Ausnehmung (101), vorzugsweise mittig, in einem scheibenförmigen Kopfbereich (110) des Führungselements (100) angeordnet ist und der Anschluss (108) als Stutzen zum Anschließen einer Schlauchleitung (111) ausgebildet ist und sich, vorzugsweise radial, in der Ebene des scheibenförmigen Bereichs (110) nach außen erstreckt.

6. Führungselement nach einem der Ansprüche 1 bis 5, **dadurch gekennzeichnet, dass** der Kanal (102) in einem ersten, dem Anschluss (108) nahen, Teilabschnitt senkrecht zur zentralen Längsachse der Ausnehmung (101) und in einem zweiten anschließenden Teilabschnitt im wesentlichen parallel zur zentralen Längsachse der Ausnehmung (101) verläuft.

7. Führungselement nach einem der Ansprüche 1 bis 6, **dadurch gekennzeichnet, dass** auf der im Betrieb der Eingangsöffnung (3) zugewendeten Seite des Führungselements (100) ein, vorzugsweise im wesentlichen zylindrischer, Vorsprung (104) ausgebildet ist, dessen Seitenfläche vorzugsweise von einem Dichtungselement (4), beispielsweise einem O-Ring, umgeben ist,
und/oder dass auf der im Betrieb der Eingangsöffnung (3) zugewendeten Seite des Führungselements (100) eine unten offene sich verjüngende kegelstumpfmantelförmige Hülse (105) ausgebildet ist, die zentral von der Ausnehmung (101) durchsetzt ist.

8. Führungselement nach Anspruch 7, **dadurch gekennzeichnet, dass** die Hülse (105) direkt an den Vorsprung (104) anschließt, wobei der größte Durchmesser der Hülse (105) vorzugsweise kleiner ist als der Durchmesser des Vorsprungs (104).

9. Führungselement nach einem der Ansprüche 1 bis 8, **dadurch gekennzeichnet, dass** die im Betrieb der Eingangsöffnung (3) zugewendete Unterfläche (106) des Führungselements (100) glatt und planeben ausgebildet ist,
und/oder dass ein seitlich vom Führungselement (100) vorstehendes Rastelement (107) vorgesehen ist, das in eine korrespondierende Rastausnehmung (115) der Vorrichtung reversibel einklickbar ist.

10. Vorrichtung zur durchdringenden Verlängerung einer in hartes Gewebe, insbesondere in den Kieferknochen (24), eingebrachten Sackbohrung, umfassend ein Arbeitswerkzeug (6), z.B. einen Fräser, das Führungselement (100) nach einem der Ansprüche 1 bis 9 sowie einen in die Sackbohrung dicht einsetzbaren, eine Druckkammer (7) ausbildenden, Hohlkörper (1) mit einem, vorzugsweise zylindrischen, Hohlraum (12), einer distalen, im Betrieb dem Knochen (24) nahen, Arbeitsöffnung (2) und einer der Arbeitsöffnung (2) gegenüberliegenden Eingangsöffnung (3), wobei die Eingangsöffnung (3) mit dem Führungselement (100) verschlossen ist, wobei ein Schaft (5) des Arbeitswerkzeugs (6) durch die Ausnehmung (101) durchführbar und in den Hohlkörper (1) einführbar ist, und wobei am Anschluss (108) des Führungselements (100) eine Schlauchleitung (111) für ein Arbeitsmedium zum Aufbringen eines Innendruckes in der Druckkammer (7) anschließbar ist.

11. Vorrichtung nach Anspruch 10, **dadurch gekennzeichnet, dass** das Führungselement (100) die Eingangsöffnung (3) und damit die Druckkammer (7) druckdicht und im wesentlichen fluiddicht verschließt und im Inneren der Druckkammer (7) vorzugsweise ein Druck von mindestens 1,5 bar, vorzugsweise mindestens 2,5 bar, erreichbar ist,
und/oder dass der Schaft (5) in der Ausnehmung (101) druckdicht und im wesentlichen fluiddicht gelagert und geführt ist und im Inneren der Druckkammer (7) vorzugsweise ein Druck von mindestens 1,5 bar, vorzugsweise mindestens 2,5 bar, erreichbar ist und gleichzeitig zumindest eine Vorschub-, Antriebs- und/oder Steuerbewegung des Schafts (5), beispielsweise eine Rotation, eine kreisende oder taumelnde Bewegung und/oder ein axialer Vorschub des Schafts (5), gewährleistet ist.

12. Vorrichtung nach einem der Ansprüche 10 bis 11, **dadurch gekennzeichnet, dass** das Führungselement (100) reversibel und zerstörungsfrei am Hohlkörper (1) befestigbar und von diesem entfernbar ist.

13. Vorrichtung nach einem der Ansprüche 10 bis 12, **dadurch gekennzeichnet, dass** am Hohlkörper (1) ein Griffelement (112) angeordnet ist.

14. Vorrichtung nach Anspruch 13, **dadurch gekennzeichnet, dass** das Führungselement (100) in einem gegenüber der verbleibenden Oberfläche vertieften Bereich (113) am distalen Ende des Griffelements (112) angeordnet ist,
und/oder dass die Schlauchleitung (111) in einer, gegebenenfalls mit Fixierelementen (117) ausgestatteten, im Griffelement (112) ausgebildeten, Nut (114) verläuft.

15. Vorrichtung nach einem der Ansprüche 10 bis 14, **dadurch gekennzeichnet, dass** eine Rastausnehmung (115), vorzugsweise in der Randfläche des vertieften Bereichs (113), ausgebildet ist, in die das korrespondierende Rastelement (107) des Führungselements (100) reversibel einklickbar und das Führungselement (100) lagefixierbar ist, wobei die Wirkverbindung insbesondere durch eine Drehverschwenkung des Führungselements (100) um etwa 30° bis 50°, mit der Ausnehmung (101) als Drehachse, erreichbar ist, und/oder dass das Führungselement (100) mit dem Vorsprung (104) fluiddicht in die Eingangsöffnung (3), insbesondere bis zu einem vorstehenden Anschlag (119) einsetzbar ist.

16. Vorrichtung nach einem der Ansprüche 10 bis 15, **dadurch gekennzeichnet, dass** die Eingangsöffnung (3) zumindest abschnittsweise in Form eines sich von einem breiteren Anfangsbereich konisch verjüngenden trichterförmigen Hohlbereichs ausgebildet ist, und/oder dass die Außenwandung der Hülse (105) von der trichterförmigen Innenwandung der Eingangsöffnung (3) beabstandet ist,
und/oder dass an der Außenseite des Hohlkörpers (1) ein entlang des Hohlkörpers (1) verstellbarer und an diesem lösbar festlegbarer Flansch (10) vorgesehen ist, der einen, vorzugsweise konischen, zwischen Hohlkörper (1) und Sackbohrungswandung reichenden Dichtungsansatz (11) aufweist,
und/oder dass zwischen dem Führungselement (100) und dem Arbeitswerkzeug (6) ein elastischer Faltenbalg (118) angeordnet ist.

17. Vorrichtung nach einem der Ansprüche 10 bis 16, **dadurch gekennzeichnet, dass** das Arbeitswerkzeug (6) ein Arbeitswerkzeug mit einem rotierenden Schaft (5), insbesondere ein Fräser, oder ein rotationsfreies Arbeitswerkzeug, beispielsweise ein Ultraschall-Osteotom oder ein Laserschneidegerät, ist,
und/oder dass die Vorrichtung über den Anschluss (108) mit einer manuellen oder automatischen Drucksteuereinheit verbunden ist,
und/oder dass die Vorrichtung eine Einrichtung zur Erzeugung bzw. Übertragung mechanischer oder elektromagnetischer Schwingungen auf das Arbeitsmedium in der Druckkammer (7) aufweist.

18. Vorrichtung nach einem der Ansprüche 10 bis 17, **dadurch gekennzeichnet, dass** sich der Durchmesser des Schafts (5) zum Bohrkopf hin stufenförmig verringert, wobei der Teilabschnitt des Schafts (5), der im Bereich der Druckkammer (7) liegt, einen geringeren Durchmesser aufweist als der Teilabschnitt des Schafts (5), der dichtend im Bereich der Ausnehmung (101) liegt,
und/oder dass der Bohrkopf des Fräsers (6) zur Gewährleistung eines möglichst punktförmigen und kleinflächigen Durchtritts durch die Knochenplatte, eine kleinflächige Spitze mit starker Krümmung aufweist.

19. Set umfassend ein Führungselement (100) gemäß einem der Ansprüche 1 bis 9 sowie zumindest eine Schlauchleitung (111) und gegebenenfalls eine Spritze, wobei das Führungselement (100) und die Schlauchleitung (111) sowie die Spritze sterilisiert und in einem geschlossenen Behältnis, vorzugsweise einem Kunststoffbeutel, aseptisch verpackt vorliegen.

## Claims

1. A guide element (100) for the arrangement on an apparatus for the penetrating extension of a blind borehole introduced into hard tissue, particularly in the jaw bone (24), wherein this apparatus comprises a hollow body (1) that forms a pressure chamber (7), said body having a distal working opening (2) and an inlet opening (3) opposite to the working opening (2), wherein the guide element (100) is insertable with a snug fit into the inlet opening (3) and the inlet opening (3) is closable by the guide element (100), wherein the guide element (100) has a continuous recess (101) through which a shaft (5) of a working tool (6), for example, a milling cutter, is manipulable and insertable into the hollow body (1), and wherein the guide element (100) comprises a connection (108) for a working medium for applying an internal pressure in the pressure chamber (7) or in the hollow body (1), **characterised in that** an interior channel (102) that is fully enclosed on all sides is formed in the guide element (100), said channel being connected to an outlet opening (103) with a connection (108) and flowing into inlet opening (3), the channel (102) running separately from the recess (101) throughout.

2. The guide element according to claim 1, **characterised in that** the guide element (100) produces a pressure-tight and substantially fluid-tight closure of the inlet opening (3),
and/or that the recess (101) produces a pressure-tight and substantially fluid-tight mounting and guiding of the shaft (5) and simultaneously guarantees at least one feed, drive and/or control movement of the shaft (5), for example, a rotational, circular, or wobbling motion and/or an axial feed of the shaft (5).

3. The guide element according to one of claims 1 through 2, **characterised in that** a sealing element, for example a sealing O-ring, is arranged inside the recess (101) for the further sealing the shaft (5).

4. The guide element according to one of claims 1 through 2, **characterised in that** the guide element (100) is formed integrally or in one piece, especially as a sterilisable disposable part, preferably made from plastic.

5. The guide element according to one of claims 1 through 4, **characterised in that** the recess (101) is arranged, preferably centrally, in a disk-shaped head region (110) of the guide element (100), and the connection (108) is formed as a connector for connecting a hose line (111) and, preferably radially, extends outwards in the plane of the disc-shaped region (110).

6. The guide element according to one of claims 1 through 5, **characterised in that** the channel (102) in a first segment close to the connection (108) runs perpendicular to the central longitudinal axis of the recess (101) and in a second subsequent segment runs substantially parallel to the central longitudinal axis of the recess (101).

7. The guide element according to one of claims 1 through 6, **characterised in that** a preferably substantially cylindrical projection (104) is formed on the side of the guide element (100) facing the input opening (3) during the operation thereof, the side surface of said projection being preferably surrounded by a sealing element (4), for example an O-ring,
and/or that a downwardly open tapered truncated conical sleeve (105) is formed on the side of the guide element (100) facing the input opening (3) during the operation thereof, which sleeve centrally interpenetrates the recess (101).

8. The guide element according to claim 7, **characterised in that** the sleeve (105) is directly connected to the projection (104), wherein the largest diameter of the sleeve (105) is preferably smaller than the diameter of the projection (104).

9. The guide element according to one of claims 1 through 8, **characterised in that** the undersurface (106) of the guide element (100) that faces towards the inlet opening (3) during the operation thereof is formed to be smooth and level,
and/or that a latching element (107) projecting laterally from the guide element (100) is provided which can reversibly be clicked into a corresponding latching recess (115) of the apparatus.

10. An apparatus for the penetrating extension of a blind borehole introduced into hard tissue, particularly in the jaw bone (24), comprising a working tool (6), for example, a milling cutter, the guide element (100) according to one of claims 1 through 9, and a hollow body (1) that is tightly insertable into the blind borehole, said body forming a pressure chamber (7), with a preferably cylindrical hollow space (12), said body having a distal working opening (2) close to the bone (24) during operation, and an inlet opening (3) opposite to the working opening (2), wherein the inlet opening (3) is closable by the guide element (100), wherein a shaft (5) of the working tool (6) is manipulable through the recess (101) and insertable into the hollow body (1), and wherein at the connection (108) of the guide element (100), a hose line (111) for a working medium can be connected for applying an internal pressure in the pressure chamber (7).

11. The apparatus according to claim 10, **characterised in that** the guide element (100) closes the inlet opening (3) and thus the pressure chamber (7) in a pressure-tight and substantially fluid-tight manner, and preferably a pressure of at least 1.5 bar, preferably at least 2.5 bar, is achievable in the interior of the pressure chamber (7), and/or that the shaft (5) is embedded and guided in the recess (101) in a pressure-tight and substantially fluid-tight manner, and preferably a pressure of at least 1.5 bar, preferably at least 2.5 bar, is achievable in the interior of the pressure chamber (7) and simultaneously at least one feed, drive and/or control movement of the shaft (5), for example, a rotational, circular, or wobbling motion and/or an axial feed of the shaft (5) is guaranteed.

12. The apparatus according to claims 10 through 11, **characterised in that** the guide element (100) is reversibly and non-destructively attachable to and removable from the hollow body (1).

13. The apparatus according to claims 10 through 12, **characterised in that** a handle element (112) is arranged on the hollow body (1).

14. The apparatus according to claim 13, **characterised in that** the guide element (100) is arranged in a recessed region (113) at the end of the handle element (112) that is distal to the remaining surface,
and/or that the hose line (111) runs in a groove (114) formed in the handle element (112), which groove is optionally equipped with fixing elements (117).

15. The apparatus according to claims 10 through 14, **characterised in that** a latching recess (115) is formed, preferably in the edge surface of the recessed region (113), into which the corresponding latching element (107) of the guide element (100) can reversibly be clicked, and the guide element (100) is positionally fixable, wherein the operative connection is achievable in particular by a rotational tilting of the guide element (100) by approx. 30° to 50°, with the recess (101) as a rotational axis,
and/or that the guide element (100) with the projection (104) is insertable into the inlet opening (3) in a fluid-tight manner, in particular up to a protruding stop (119).

16. The apparatus according to claims 10 through 15, **characterised in that** the inlet opening (3) is at least partially formed in the shape of a funnel-shaped hollow region that conically tapers down from a wider initial region,
and/or that the outer wall of the sleeve (105) is spaced apart from the funnel-shaped inner wall of the inlet opening (3),
and/or that on the outside of the hollow body (1) is provided a flange (10) that is adjustable along the length of the hollow body (1) and releasably fixable thereto, which flange has a preferably conical sealing protrusion (11) reaching between hollow body (1) and a blind borehole wall,
and/or that an elastic bellows (118) is arranged between the guide element (100) and the working tool (6).

17. The apparatus according to claims 10 through 16, **characterised in that** the working tool (6) is a working tool with a rotating shaft (5), in particular a milling cutter, or a non-rotating working tool, for example an ultrasonic osteotome or a laser cutting device,
and/or that the apparatus is connected via the connection (108) with a manual or automatic pressure control unit,
and/or that the apparatus comprises a device for the production or transmission of mechanical or electromagnetic vibrations to the working medium in the pressure chamber (7).

18. The apparatus according to claims 10 through 17, **characterised in that** the diameter of the shaft (5) is reduced stepwise toward the drill head, wherein the part of the shaft (5) which lies in the region of the pressure chamber (7) has a smaller diameter than the section of the shaft (5) which is present in a sealed fashion in the region of the recess (101),
and/or that the drill head of the milling cutter (6) has a small area tip with high curvature to ensure a most punctiform and smallest-scale passage possible through the bone plate.

19. A set comprising a guide element (100) according to any one of claims 1 through 9 and at least one hose line (111) and, optionally, a syringe, wherein the guide element (100) and the hose line (111) and the syringe are sterilised and packaged aseptically in a closed container, preferably a plastic bag.

## Revendications

1. Élément de guidage (100) à placer sur un dispositif destiné à perforer une extension de trou borgne dans un tissu dur, en particulier dans l'os de la mâchoire (24), ledit dispositif comprenant un corps creux (1) formant une chambre de pression (7) présentant une ouverture de travail distale (2) et une ouverture d'entrée (3) face à l'ouverture de travail (2), ledit élément de guidage (100) pouvant être inséré avec un ajustement fin dans l'ouverture d'entrée (3) et l'ouverture d'entrée (3) pouvant être fermée par ledit élément de guidage (100), ledit élément de guidage (100) présentant un évidement transversal (101), à travers lequel peut passer une tige (5) d'un outil de travail (6), par exemple d'une fraiseuse, et pouvant être insérée dans le corps creux (1) et ledit élément de guidage (100) présentant un raccord (108) pour un milieu de travail destiné à appliquer une pression interne dans la chambre de pression (7), respectivement dans le corps creux (1), **caractérisé en ce que**
un canal interne fermé de tous côtés (102) est formé dans l'élément de guidage (100), ledit canal interne reliant le raccord (108) à une ouverture de sortie (103) qui débouche dans l'ouverture d'entrée (3) et **en ce que** le canal (102) s'étend à partir de l'évidement (101) généralement distinct.

2. Élément de guidage selon la revendication 1, **caractérisé en ce que** l'élément de guidage (100) ferme l'ouverture d'entrée (3) de manière étanche à la pression et à la plupart des fluides,
et/ou **en ce que** l'évidement (101) permet la mise en place et le guidage de la tige (5) de manière étanche à la pression et à la plupart des fluides et garantit dans le même temps au moins un mouvement d'avancée, d'entraînement et/ou de commande de la tige (5), par exemple une rotation, un mouvement circulaire, respectivement de va-et-vient, et/ou une avancée axiale de la tige (5).

3. Élément de guidage selon l'une des revendications 1 à 2, **caractérisé en ce qu'**un élément d'étanchéité, par exemple un joint torique, est disposé à l'intérieur de l'évidement (101), afin de renforcer l'étanchéité de la tige (5).

4. Élément de guidage selon l'une des revendications 1 à 2, **caractérisé en ce que** ledit élément de guidage (100) est construit d'un seul morceau, respectivement d'une seule pièce, en particulier sous la forme d'un élément de construction jetable stérilisable, de préférence en plastique.

5. Élément de guidage selon l'une des revendications 1 à 4, **caractérisé en ce que** l'évidement (101) est ménagé au milieu de l'élément de guidage (100), au niveau d'une partie tête (110) en forme de disque, et le raccord (108) est sous la forme d'un manchon destiné à être relié à un tuyau (111) et s'étend vers l'extérieur, de préférence de manière radiale, à proximité de la partie (110) en forme de disque.

6. Élément de guidage selon l'une des revendications 1 à 5, **caractérisé en ce que** le canal (102) s'étend dans une première section à proximité du raccord (108) perpendiculairement à l'axe longitudinal central de l'évidement (101) et dans une seconde section de raccordement essentiellement parallèle à l'axe longitudinal central de l'évidement (101).

7. Élément de guidage selon l'une des revendications 1 à 6, **caractérisé en ce qu'**une saillie (104), de préférence essentiellement cylindrique, est formée sur le côté de l'élément de guidage (100) qui est orienté dans la direction de l'ouverture d'entrée (3), dont la surface latérale est de préférence entourée par un élément d'étanchéité (4), par exemple un joint torique,
et/ou **en ce que** l'élément de guidage (100), qui est orienté dans la direction de l'ouverture d'entrée (3), est formé d'une douille (105) tronconique biseautée ouverte vers le bas, laquelle est introduite au centre de l'évidement (101).

8. Élément de guidage selon la revendication 7, **caractérisé en ce que** la douille (105) est directement raccordée à la saillie (104), le diamètre maximal de la douille (105) étant de préférence inférieur au diamètre de la saillie (104).

9. Élément de guidage selon l'une des revendications 1 à 8, **caractérisé en ce que** la surface inférieure (106) orientée en direction de l'ouverture d'entrée (3) de l'élément de guidage (100) a un aspect lisse et plat,
et/ou **en ce qu'**un élément d'encliquetage (107) est disposé latéralement par rapport à l'élément de guidage (100), lequel peut être clipsé de manière réversible dans un évidement d'encliquetage (115) correspondant du dispositif.

10. Dispositif destiné à perforer une extension de trou borgne dans un tissu dur, en particulier dans l'os de la mâchoire (24), comprenant un outil de travail (6), par exemple une fraiseuse, l'élément de guidage (100) selon l'une des revendications 1 à 9, ainsi qu'un corps creux (1) formant une chambre de pression (7) pouvant être insérée de manière étanche dans le trou borgne, le corps creux (1) comprenant un espace creux (12) essentiellement cylindrique, une ouverture de travail distale (2) proche de la zone de l'os (24), et une ouverture d'entrée (3) face à l'ouverture de travail (2), ladite ouverture d'entrée (3) étant fermée par l'élément de guidage (100), une tige (5) de l'outil de travail (6) pouvant passer à travers l'évidement (101) et pouvant être insérée dans le corps creux (1), et un tuyau (111) pour un milieu de travail destiné à appliquer une pression interne dans la chambre de pression (7) pouvant être raccordé au raccord (108) de l'élément de guidage (100).

11. Dispositif selon la revendication 10, **caractérisé en ce que** l'élément de guidage (100) ferme l'ouverture d'entrée (3), et par conséquent la chambre de pression (7), de manière étanche à la pression et à la plupart des fluides, et la pression à l'intérieur de la chambre de pression (7) pouvant atteindre au moins 1,5 bar, de préférence au moins 2,5 bar,
et/ou **en ce que** la tige (5) est mise en place et guidée dans l'évidement (101) de manière étanche à la pression et à la plupart des fluides et la pression à l'intérieur de la chambre de pression (7) pouvant atteindre au moins 1,5 bar, de préférence au moins 2,5 bar, et ceci garantissant dans le même temps au moins un mouvement d'avancée, d'entraînement et/ou de commande de la tige (5), par exemple une rotation, un mouvement circulaire, ou de va-et-vient, et/ou une avancée axiale de la tige (5).

12. Dispositif selon l'une des revendications 10 à 11, **caractérisé en ce que** l'élément de guidage (100) peut être fixé au corps creux (1), et retiré de celui-ci, de manière réversible et non destructive.

13. Dispositif selon l'une des revendications 10 à 12, **caractérisé en ce qu'**un élément poignée (112) est disposé sur le corps creux (1).

14. Dispositif selon la revendication 13, **caractérisé en ce que** l'élément de guidage (100) est disposé à l'extrémité distale de l'élément poignée (112) dans une zone évidée (113) faisant face à la surface restante,
et/ou **en ce que** le tuyau (111) s'étend dans un enfoncement (114), éventuellement équipé d'éléments de fixation (117), ménagé dans l'élément poignée (112).

15. Dispositif selon l'une des revendications 10 à 14, **caractérisé en ce qu'**un évidement d'encliquetage (115) est ménagé de préférence dans la surface périphérique de la zone évidée (113), dans lequel l'élément d'encliquetage (107) correspondant de l'élément de guidage (100) peut être clipsé de manière réversible, et l'élément de guidage (100) pouvant être fixé en position, la liaison fonctionnelle étant assurée en particulier par un pivotement de l'élément de guidage (100) d'environ 30 ° à 50 °, autour de l'axe de l'évidement (101),
et/ou **en ce que** l'élément de guidage (100) comprenant la saillie (104) peut être mis en place de manière étanche aux fluides dans l'ouverture d'entrée (3), en particulier jusqu'à une butée (119).

16. Dispositif selon l'une des revendications 10 à 15, **caractérisé en ce que** l'ouverture d'entrée (3) est construite à partir d'une zone plus large au moins partiellement sous forme d'une zone creuse conique biseautée en forme d'entonnoir, et/ou **en ce que** la paroi extérieure de la douille (105) est éloignée de la paroi intérieure en forme d'entonnoir de l'ouverture d'entrée (3),
et/ou **en ce qu'**un rebord (10) ajustable le long du corps creux (1) et fixé de manière amovible à celui-ci est prévu du côté extérieur du corps creux (1), ledit rebord présentant un joint (11) de préférence conique entre le corps creux (1) et atteignant la paroi du trou borgne,
et/ou **en ce qu'**un soufflet élastique (118) est disposé entre l'élément de guidage (100) et l'outil de travail (6).

17. Dispositif selon l'une des revendications 10 à 16, **caractérisé en ce que** l'outil de travail (6) est un outil de travail comprenant une tige rotative (5), en particulier une fraiseuse, ou un outil de travail non tournant, par exemple un ostéotome à ultrasons ou un instrument de découpe laser,
et/ou **en ce que** le dispositif est relié à une unité de mise sous pression manuelle ou automatique par le biais du raccord (108),
et/ou **en ce que** le dispositif comprend un équipement destiné à la production, respectivement à la diffusion d'ondes électromagnétiques sur le milieu de travail présent dans la chambre de pression (7).

18. Dispositif selon l'une des revendications 10 à 17, caractérisé en ce le diamètre de la tige (5) diminue graduellement dans la direction de la tête de forage, la section de la tige (5) située dans la zone de la chambre de pression (7) présentant un diamètre plus faible que la section de la tige (5) disposée de manière étanche dans la zone de l'évidement (101),
et/ou en ce que la tête de forage de la fraiseuse (6) présente une pointe de faible surface et de forte courbure, afin de garantir un passage aussi ponctuel et de faible surface que possible à travers la plaque osseuse.

19. Ensemble comprenant un élément de guidage (100) selon l'une des revendications 1 à 9, ainsi qu'un tuyau (111) et éventuellement une seringue, l'élément de guidage (100) et le tuyau (111) ainsi que la seringue étant stérilisés et emballés de manière aseptique dans un récipient fermé, de préférence une poche en plastique.
